# EUROPÄISCHE PATENTSCHRIFT

(11) **EP 1 049 429 B1**
(45) Veröffentlichungstag und Bekanntmachung des Hinweises auf die Patenterteilung: **20.04.2005**
(21) Anmeldenummer: 99904781.4
(22) Anmeldetag: 15.01.1999
(51) Int. Cl.: A61F 2/40

(54) **HUMERUSKOPFPROTHESE**
HUMERAL HEAD PROSTHESIS
PROTHESE DE TETE D'HUMERUS

(30) Priorität: 22.01.1998 DE 29800975 U
(43) Veröffentlichungstag der Anmeldung: 08.11.2000
(73) Patentinhaber: Zimmer GmbH, 8404 Winterthur (CH)
(72) Erfinder: Rüter, Axel, 86356 Neusäss (DE)
(74) Vertreter: Manitz, Finsterwald & Partner Gbr
(86) Internationale Anmeldenummer: PCT/EP1999/000209
(87) Internationale Veröffentlichungsnummer: WO 1999/037254

(56) Entgegenhaltungen:
- EP-A- 0 191 182
- EP-A- 0 423 064
- EP-A- 0 466 638
- WO-A-96/36300
- WO-A-97/39693
- DE-A- 19 614 949
- FR-A- 2 726 994

## Beschreibung

Die Erfindung zeigt eine Humeruskopfprothese mit den Merkmalen im Oberbegriff des Hauptanspruchs.

Eine solche Humeruskopfprothese oder Schulterprothese ist aus dem Stand der Technik bekannt, z.B. aus WO-A-97/39693. Sie besitzt einen Kopf und einen Schaft. Der im wesentlichen einem Kugelsegment entsprechende Kopf kann mit dem Schaft lösbar über einen Hals verbunden sein. Er weist für den Hals eine exzentrische Bohrung auf. Bei Humeruskopfprothesen besteht das Problem der knöchernen Integration der Tuberkula an der Prothese. Die vorbekannte Humeruskopfprothese bietet hierfür keine praktikable und sichere Hilfe an.

Es ist daher Aufgabe der vorliegenden Erfindung, eine Humeruskopfprothese mit einer besseren Integrationsmöglichkeit der Tuberkula aufzuzeigen.

Die Erfindung löst diese Aufgabe mit den Merkmalen im Hauptanspruch. Der erfindungsgemäß am Schaft angeordnete hohle Köcher erleichtert die Integration der Tuberkula an der Prothese. Er kann mit Knochenspänen oder Knochensplittern gefüllt werden, wobei durch die Mantelöffnungen eine Ossifikation und Verbindung mit den oder der Tuberkula möglich ist. In vielen Fällen ist bei einer Schulterverletzung die Sehne an den Tuberkula noch angewachsen, wobei die Ablösung im Knochen stattgefunden hat. Die Sehnen können dann mit den Tuberkula wieder an der Humeruskopfprothese befestigt und über die Ossifikation integriert werden.

Die Mantelöffnungen des Köchers bieten außerdem eine Möglichkeit zur exakten Positionierung und temporären Befestigung der Tuberkula an der anatomisch richtigen Stelle. Zu diesem Zweck ist es auch erfindungsgemäß vorgesehen, daß der Köcher subkapital in Höhe der anatomischen Stelle der Tuberkula und lateral am Schaft angeordnet ist. Die proximal gelegene Einfüllöffnung erleichtert das Einbringen der Knochenspäne. Die trichterartige Köcherform kommt dem ebenfalls entgegen, wobei die Trichterform außerdem die anatomisch korrekte Positionierung der Tuberkula unterstützt und zudem in anatomisch günstiger Weise im proximalen Bereich mehr Knochenmaterial zum Anwachsen bietet als im distalen Bereich.

In besonders vorteilhafter Weise sind zumindest ein Teil der Mantelöffnungen mit Schraubgewinden versehen, die eine exakte Positionierung der Tuberkula mittels Knochenschrauben gestatten. Zur Verbesserung der Wirtschaftlichkeit ist es dabei günstig, den Gewindebohrungen ein metrisches Gewinde zu geben, um kostengünstige Standardschrauben mit Preßkragen verwenden zu können.

In den Unteransprüchen sind weitere vorteilhafte Ausgestaltungen der Erfindung angegeben.

Die Erfindung ist in den Zeichnungen beispielsweise und schematisch dargestellt. Im einzelnen zeigen:

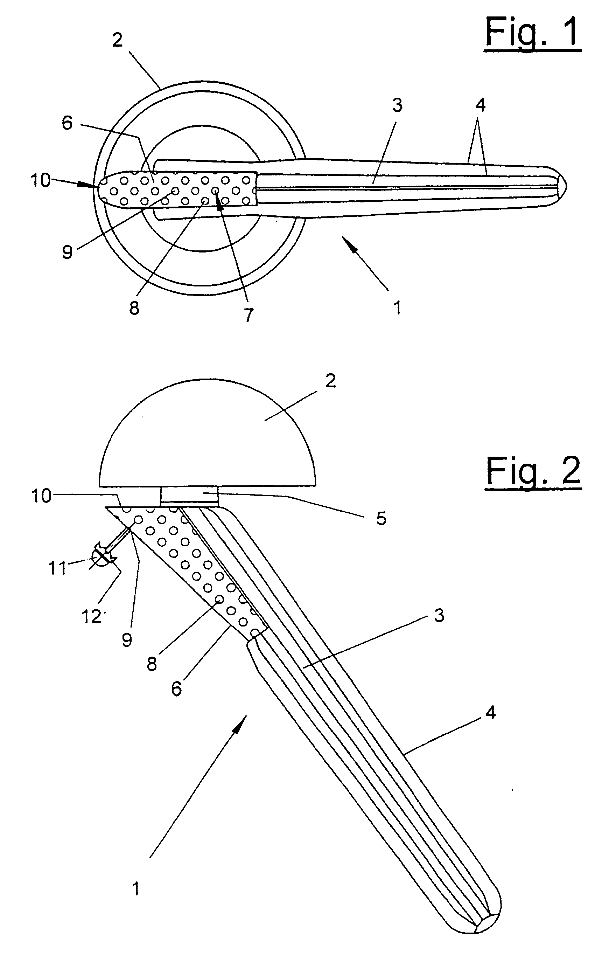

- Figur 1:: eine Humeruskopfprothese in der Unteransicht vom distalen Ende aus gesehen und
- Figur 2:: eine Seitenansicht der Prothese von Figur 1.

Die Humeruskopfprothese (1) besteht aus einem im wesentlichen halbkugelförmigen Kopf (2) und einem Schaft (3), die über einen Hals (5) verbunden sind. Die Verbindung zwischen dem Hals (5) und dem Kopf (2) kann lösbar sein. Die Humeruskopfprothese (1) kann im Schaftund Kopfbereich eine beliebig geeignete Formgebung besitzen.

Am Prothesenschaft (3) ist lateral ein hohler Köcher (6) zur Aufnahme von Knochenspänen, Knochensplittern oder dergleichen angeordnet. Der Köcher (6) befindet sich subkapital in Höhe der anatomischen Stelle der Tuberkula und schließt vorzugsweise am proximalen Ende bündig mit dem Prothesenschaft (3) ab. Im Köcherbereich kann der Prothesenschaft (3) eine Abflachung oder Ausnehmung aufweisen. Dies erleichtert zum einen das Anbringen des Köchers (6) und vergrößert zum anderen das Köchervolumen.

Die Humeruskopfprothese (1) und der Köcher (6) bestehen aus einem körperfreundlichen inerten Material, z. B. Titan. Der Köcher (6) hat eine gewölbte Form und ist mit seinen Rändern am Schaft (3) in geeigneter Weise verbunden, z. B. durch Schweißen oder dergleichen. Hierbei können Führungen, Zentrierungen und andere Positionierund Befestigungshilfen vorhanden sein.

Der hohle Köcher (6) hat eine nach proximal sich erweiternde Trichterform und besitzt proximal eine Einfüllöffnung (10). Hierdurch können die Knochenspäne von proximal her eingefüllt werden. Der Kopf (2) kann dazu vom Hals (5) abgenommen werden. Ein Einfüllen ist gegebenenfalls auch unter dem angesetzten Kopf möglich.

Der Köcher hat einen Mantel mit ein oder mehreren Öffnungen (8). Diese können kreisrund sein und eine andere beliebige Form haben und aus dem Mantel freigestanzt oder in sonstiger geeigneter Weise hergestellt sein. Der Köchermantel kann ferner ein oder mehrere Gewindebohrungen (9) besitzen. Hierbei können ein Teil oder alle der Öffnungen (8) als Gewindebohrungen (9) ausgebildet sein. Vorzugsweise befinden sich die Gewindebohrungen (9) im lateralen Mantelbereich.

Die Gewindebohrungen haben ein metrisches Gewinde und können Standardschrauben (11) aufnehmen. Ansonsten können aber auch andere Arten von Schrauben mit anderen Gewinden verwendet werden. Es ist auch möglich, Schrauben mit selbstschneidenden Gewinden oder Blechgewinden zur Verbindung mit einfachen Öffnungen (8) zu verwenden.

In der bevorzugten Ausführungsform haben die Schrauben (11) außerdem einen Preßkragen (12). Der topfartige Preßkragen (12) hat randseitig vorstehende Spitzen, die den Anpreßdruck besser verteilen und für einen sicheren Schraubenhalt sorgen.

Die erfindungsgemäße Humeruskopfprothese (1) ermöglicht die exakte Integration abgelöster Tuberkula (nicht dargestellt). Dazu werden ein oder mehrere Schrauben (11) über geeignete Durchgangsbohrungen in den Tuberkula gesteckt und an den Öffnungen (8) bzw. Gewindebohrungen (9) des Köchers (6) fixiert.

Die Humeruskopfprothese (1) kann ansonsten noch beliebige andere Gestaltungsmerkmale haben. Der Schaft (3) besitzt z.B. ein oder mehrere längslaufende Rippen (4) zur Fixierung im Humerus. Der Kopf (2) kann eine exzentrische Aufnahmeöffnung für den Hals (5) aufweisen. Außerdem kann er zur Materialeinsparung Ausdrehungen und andere Ausnehmungen haben.

Abwandlungen der gezeigten Ausführungsform sind in verschiedener Weise möglich. So kann der hohle Köcher eine beliebig andere geeignete Form und Lage am Prothesenschaft (3) haben. Außerdem können die Tuberkula statt durch Schrauben auch auf andere geeignete Weise am Köcher (6) befestigt werden. Die Öffnungen (8) bzw. Gewindebohrungen (9) können am Köchermantel in der gezeigten Form gleichmäßig oder ungleichmäßig verteilt sein. Ihre Zahl und Anordnung kann beliebig variieren.

### BEZUGSZEICHENLISTE

- 1: Humeruskopfprothese
- 2: Kopf
- 3: Schaft
- 4: Rippe
- 5: Hals
- 6: Köcher
- 7: Innenraum
- 8: Öffnungen
- 9: Gewindebohrung
- 10: Einfüllöffnung
- 11: Schraube, Standardschraube
- 12: Preßkragen

## Patentansprüche

1. Humeruskopfprothese mit einem Kopf (2) und einem Schaft (3), wobei proximal und lateral am Schaft (3) ein hohler Köchen (6) angeordnet ist, **dadurch gekennzeichnet, dass** der köcher mit einem eine oder mehrere Öffnungen (8) aufweisenden Mantel versehen ist und der Köcher (6) subkapital in Höhe der anatomischen Stelle der Tuberkula am Schaft (3) angeordnet ist.

2. Humeruskopfprothese nach Anspruch 1, **dadurch gekennzeichnet, daß** der Köcher (6) proximal eine Einfüllöffnung (10) aufweist.

3. Humeruskopfprothese nach Anspruch 1 oder 2, **dadurch gekennzeichnet, daß** der Köcher (6) eine nach proximal sich erweiternde Trichterform aufweist.

4. Humeruskopfprothese nach einem der Ansprüche 1 bis 3, **dadurch gekennzeichnet, daß** der Köcher (6) ein oder mehrere Gewindebohrungen (9) im Mantel aufweist.

5. Humeruskopfprothese nach Anspruch 4, **dadurch gekennzeichnet, daß** die Gewindebohrungen (9) ein metrisches Gewinde zur Aufnahme von Standardschrauben (11) mit Preßkragen (12) aufweisen.

## Claims

1. Humerus head prosthesis comprising a head (2) and a shaft (3), wherein a hollow quiver (6) is arranged proximally and laterally at the shaft (3), **characterized in that** the quiver is provided with a jacket having one or more openings (8); and **in that** the quiver (6) is arranged at the shaft (3) subcapitally at the level of the anatomical location of the tubercles.

2. Humerus head prosthesis in accordance with claim 1, **characterized in that** the quiver (6) has a filling opening (10) proximally.

3. Humerus head prosthesis in accordance with claim 1 or claim 2, **characterized in that** the quiver (6) has a funnel shape which diverges towards proximal.

4. Humerus head prosthesis in accordance with any one of the claims 1 to 3, **characterized in that** the quiver (6) has one or more threaded bores (9) in the jacket.

5. Humerus head prosthesis in accordance with claim 4, **characterized in that** the threaded bores (9) have a metric thread for the reception of standard screws (11) with compression collars (12).

## Revendications

1. Prothèse de tête d'humérus avec une tête (2) et une tige (3), un carquois creux (6) étant disposé au niveau proximal et latéralement sur la tige (3), **caractérisée en ce que** le carquois est muni d'une enveloppe comportant un ou plusieurs orifices (8), et **en ce que** le carquois (6) est disposé de façon sub-capitale sur la tige (3) à hauteur de l'emplacement anatomique du tubercule.

2. Prothèse de tête d'humérus selon la revendication 1, **caractérisée en ce que** le carquois (6) comporte un orifice de remplissage (10) au niveau proximal.

3. Prothèse de tête d'humérus selon la revendication 1 ou 2, **caractérisée en ce que** le carquois (6) possède une forme d'entonnoir s'élargissant dans le sens proximal.

4. Prothèse de tête d'humérus selon l'une quelconque des revendications 1 à 3, **caractérisée en ce que** le carquois (6) comporte un ou plusieurs taraudages (9) dans l'enveloppe.

5. Prothèse de tête d'humérus selon la revendication 4, **caractérisée en ce que** les taraudages (9) comportent un filetage métrique pour la réception de vis standard (11) à collerette de pression (12).
